# EUROPEAN PATENT APPLICATION

(11) **EP 4 385 392 A1**
(43) Date of publication of application: **19.06.2024**
(21) Application number: 22213696.2
(22) Date of filing: 15.12.2022
(51) Int. Cl.: A61B 5/00

(54) **A SYSTEM AND METHOD FOR IMAGING TISSUE**

(71) Applicant: Koninklijke Philips N.V., 5656 AG Eindhoven (NL)
(72) Inventor: DAMODARAN, Mathivanan, Eindhoven (NL); PAULUSSEN, Elvira Johanna Maria, Eindhoven (NL); JOHNSON, Mark Thomas, Eindhoven (NL); GERHARDT, Lutz Christian, Eindhoven (NL); SEGAAR, ucja El bieta, Eindhoven (NL)
(74) Representative: Philips Intellectual Property & Standards

(57) **Abstract**

A system is provided for imaging a tissue during application of a variable pressure to the tissue which induces tissue deformation or movement. An optical imaging system comprises a light source, static optics, and at least one light detector. A distance between the optical imaging system and the tissue is variable in dependence on the tissue deformation or movement such that different portions of the tissue lie within the depth of field of the imaging system at different times during the application of the variable pressure. The captured images are processed to construct a combined image which includes said different portions of the tissue. In other words, instead of scanning an optical imaging system over an area or volume of tissue, pressure-induced movement of the tissue is used to provide an alternative to scanning of the imaging system.

## Description

### FIELD OF THE INVENTION

This invention relates to the imaging of tissue.

### BACKGROUND OF THE INVENTION

There are many situations in which imaging of tissue is desirable. Low-cost tissue imaging systems make use of optical image sensors (rather than ultrasound, X-ray or MRI imaging for example), and low-cost tissue imaging may be incorporated into personal care devices for consumer use, for various purposes.

One example of interest is imaging of breast tissue. Breastfeeding is crucial for the healthy development of a newborn baby. Indeed, the best source of nutrition for a baby is human milk given by a breastfeeding mother. The milk contains antibodies which help protect against many common childhood and future illnesses. The World Health Organization recommends exclusively breastfeeding for at least six months, meaning no other foods or liquids are provided.

During breastfeeding, the infant interacts with the nipple by applying both vacuum cycles and tongue pressures to extract milk. Moreover, the infant stimulates the nipple and areola complex that leads to release of oxytocin and ejection of milk. The combination of the applied vacuum, the contracting response of the cells around the ducts (myoepithelial cells) to oxytocin release and the pressure of milk flowing through the ducts leads to opening of the milk ducts and release of milk.

In this process, the milk ducts show a highly dynamic behavior, going from a collapsed to a swollen milk filled state. Ultrasound imaging studies have shown that the milk duct size is highly responsive to pressure fluctuations (which also happens during the use of breast pumps).

However, breastfeeding is often halted due to breastfeeding related breast pain and breast traumas caused by the act of breastfeeding and/or milk expression itself. Pain and trauma are among the main causes of weaning from breastfeeding.

For example, mastitis is a common condition occurring during breastfeeding, being a painful inflammation of the breast tissue. It is most often caused by clogged milk ducts that lead to milk stasis (remaining milk in the breast after a feed giving a restricted milk flow), sometimes in combination with infectious bacteria. Clogged milk ducts refer to milk ducts with some obstruction, e.g. caused by milk clutters. They can be painful, feel warm, cause discomfort and as stated above lead to the development of mastitis.

Clogged milk ducts can be released by applying massage, warmth, or breastfeeding and/or pumping on a more frequent basis to open blocked ducts. Some signs can be predictive of clogged ducts, including localized redness of the skin, a hard or semi-hard lump on the breast, and a feeling of pain during milk ejection reflex.

However, due to the various symptoms which can result from breastfeeding (like damaged nipple, blebs, abscesses and more) mothers find it difficult to recognize and identify the right condition and, as consequence, do not timely apply the most suitable treatment preventing the development of more serious conditions and eventually, weaning from breastfeeding.

In clinical breastfeeding research, ultrasound-based imaging technologies are used to image milk ducts. This imaging method would be difficult to translate to a home solution (as mentioned above), in particular since ultrasound requires a coupling between the ultrasound transducer and the tissue, e.g., using an ultrasound gel, which is inconvenient and also not desirable as it may end up in the milk. Also, ultrasound does not have a high enough resolution to see narrower sections of the ducts (duct endings are of the order of 0.07 mm in size).

Currently, there are no consumer-friendly solutions for early detection of clogged ducts or other conditions relating to the breast, which may have an impact on breastfeeding. The only methods relate to visual or physical judgment of the mother herself or a trained professional. Those methods, however, come often late or are very subjective.

A tissue imaging technology suitable for integration into personal care devices, such as breast pumps, would be of particular interest. One area of interest would be a technology able to detect clogged milk ducts early and which is suitable for consumer use, so the mother can clear the clogged ducts on time to prevent pain, the development of mastitis and eventually, prevent weaning from breastfeeding.

### SUMMARY OF THE INVENTION

The invention is defined by the claims.

According to examples in accordance with an aspect of the invention, there is provided a system for imaging a tissue during application of a variable pressure to the tissue which induces tissue deformation or movement, comprising:
an optical imaging system comprising a light source, static optics for illuminating the tissue and/or collecting the photons that have interacted with the tissue, and at least one light detector,
wherein a distance between the optical imaging system and the tissue is variable in dependence on the tissue deformation or movement such that different portions of the tissue lie within the depth of field of the imaging system at different times during the application of the variable pressure; and
a processor for processing images captured by the optical imaging system to construct a combined image which includes said different portions of the tissue.

There is a trend to integrate cameras into handheld and personal health devices to create value for the end users by providing insights into health status. Such imaging-assisted care devices can be an enabler for remote image-based diagnostics, superior location sensing, therapy planning or treatment monitoring.

This system makes use of an imaging system to perform a scan of an area or volume of tissue so that a larger image is built up from multiple images over time. The larger image may be the result of a 1D, 2D or 3D scanning function. However, the scanning function is implemented using static optics. By this is meant there is no optical scanning function - the field of view of the optical imaging system is the same over time. Instead, movement of the tissue caused by an applied pressure creates the desired relative movement between the tissue being imaged and the imaging system.

The different images which are captured can be stitched together using known image processing methods, in particular based on recognition and alignment of keypoints in the different images, for example keypoints which are present in multiple images.

The system is for example for imaging breast tissue. In this example, the tissue is breast tissue and the movement may result from a periodic pressure waveform applied by a breast pump. The static optics thus requires no actuation signal to perform a scanning of different tissue areas or volumes.

The system is for example able to monitor a large part or all of the nipple (surface and sub-surface) during expression without needing bulky optics with movable parts. As only a section or part of the breast tissue e.g., nipple moves into the field of view of the imaging system, line, area or volume images can be reconstructed from a sequence of multiple images acquired during movement of the breast, for example caused by the pumping action of a breast pump.

Imaging the breast tissue e.g., nipple in this way during milk expression is highly valuable for assessing nipple health, for analyzing a severity or level of nipple damage (like wounds), and for early detection of blocked milk ducts. This can enable the mother to take preventative actions to avoid pain and inflammation, the development of mastitis and eventually, prevent weaning from breastfeeding. Monitoring the nipple and its stretch while feeding or expressing can also give insights on milk ejection reflex.

The optical imaging system is for example configured to image the breast from in front of the nipple or from a side of the nipple. Thus, the movement detected may be translational movement (parallel to the axis of the nipple) or it may be lateral movement, for example caused by deformation of the shape of the breast.

In one example, the static optics for example comprises an axicon lens for generating a ring of illumination. The position of the tissue determines the size of a circle formed on the tissue surface, for example if the ring is oriented around a nipple (i.e. when imaging the breast from in front).

The optical imaging system may be adapted to generate a line profile. Multiple lines may then be combined to create a 2D area image. A lens may be used for generating a line of illumination and the light detector is then a line detector.

In another example, the static optics comprises a lens for generating a focused illumination spot and the light detector is for detecting scattered, reflected light. This provides epi-illumination.

In another example, the static optics may comprise a lens for generating a focused illumination spot and the light detector is for detecting light which has being transmitted through the full volume of the tissue. This provides trans-illumination.

The optical imaging system may further comprise of at least one polarizer. This can be used in illumination and/or detection to remove surface glare and specular reflection of the tissue and to capture scattered photons and sub-surface features more deeply. This may be used in trans-illumination or epi-illumination.

The optical imaging system may further comprise one ore more fluorescence filters. This may be used to capture spectral properties of the tissue (surface and sub-surface). This may be used in trans-illumination or epi-illumination.

The light source may be a broadband light source or a wavelength sweeping source, and the detector may be a hyperspectral detector or a single point detector. This may be used in trans-illumination or epi-illumination.

The processor is for example for processing captured images from different angular orientations relative to the tissue to create a 2D or 3D volumetric scan. The rotation around the tissue is for example implemented manually. Alternatively, a mechanical system may rotate the optical imaging system. For example, a pumping action, e.g. of a breast pump, may implement an incremental rotation of the optical imaging system.

The processor may be further configured to determine elastic properties of the nipple, for example by optical coherence tomographic (OCT) elastography and/or provide Doppler flow imaging for example by Doppler OCT.

The invention also provides a breast pump comprising:
a pressure source for generating a cyclic pressure; and
a breast shield for applying the cyclic pressure to the breast; and
the tissue imaging system as defined above for detecting milk duct blockage.

The invention also provides a method for imaging a tissue, comprising:
illuminating the tissue and detecting photons that have interacted with the tissue using an optical imaging system thereby to generate captured images;
applying a variable pressure to the tissue which induces tissue deformation or movement, wherein a distance between the optical imaging system and the tissue is variable in dependence on the tissue deformation or movement such that different portions of the tissue lie within the depth of field of the imaging system at different times during the application of the variable pressure; and
processing images captured by the optical imaging system to construct a combined image which includes said different portions of the tissue.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows an optical coherence tomography, OCT, system which may be used by the tissue imaging system of the invention;
Fig. 2 is a schematic diagram of a Spectral Domain-OCT system applied to a tissue analysis system for imaging breast tissue;
Fig. 3 shows the received interference signal at the linear array detector;
Fig. 4 shows one general arrangement with the light source and detector on opposite lateral sides of the nipple;
Fig. 5 shows another general arrangement with the light source and detector on the same lateral side of the nipple;
Fig. 6 shows another general arrangement with the light source and detector in front of the nipple;
Fig. 7 shows a first example of static optics comprising an axicon lens to create a ring of illumination;
Fig. 8 shows the depth of focus of an axicon lens;
Fig. 9 shows how the detection signal of a 1D (point) detector may vary over time in synchronism with a breast pumping action;
Fig. 10 shows that polarization optics may be used in the illumination and detection;
Fig. 11 shows that a fluorescence filter may be provided in front of the detection optics; and
Fig. 12 shows a known breast pump system which may incorporate the tissue imaging system.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a system for imaging a tissue during application of a variable pressure to tissue which induces tissue deformation or movement. An optical imaging system comprises a light source, static optics, and at least one light detector. A distance between the optical imaging system and the tissue is variable in dependence on the tissue deformation or movement such that different portions of the tissue lie within the depth of field of the imaging system at different times during the application of the variable pressure. The captured images are processed to construct a combined image which includes said different portions of the tissue. In other words, instead of scanning an optical imaging system over an area or volume of tissue, pressure-induced movement of the tissue is used to provide an alternative to scanning of the imaging system.

As outlined above, the invention makes use of optical imaging.

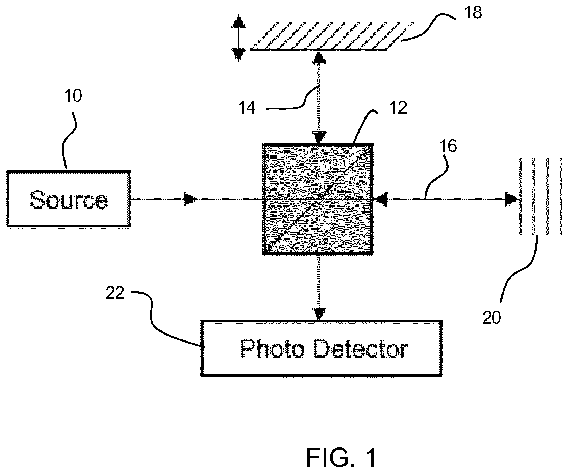

Fig. 1 shows an optical coherence tomography, OCT, system which may be used by the tissue imaging system of the invention.

OCT uses a standard Michelson Interferometer with a low-coherence light source 10 as shown in Fig. 1. In the interferometer, the incoming broadband beam of light is split by a splitter 12 into a reference path 14 and a sample path 16 which are recombined after back-reflection from the reference mirror 18 and the multiple layers of the sample 20, respectively, to form an interference signal, which is detected by a photodetector 22.

The broadband nature of light causes interference of the optical fields to occur only when the path lengths of the reference and the sample arm are matched within the coherence length of the light. This interference signal carries information about the sample at a depth determined by the reference path length.

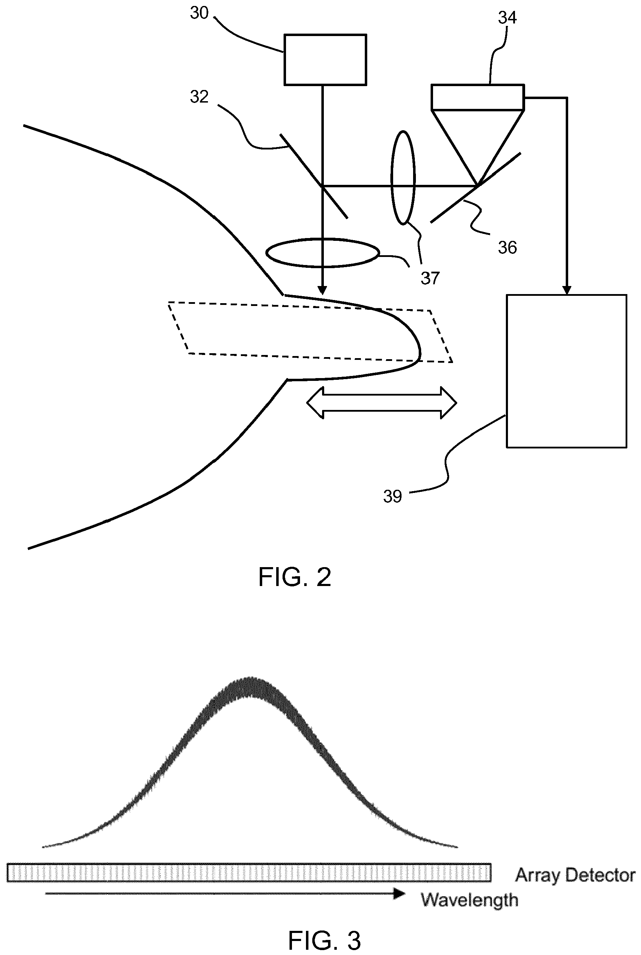

Fig. 2 represents a schematic diagram of the Spectral Domain-OCT (SD-OCT) system applied to a tissue analysis system for imaging breast tissue, in particular for imaging the nipple to provide images which can be used to detect milk duct condition.

A broadband-source of light 30 with a short temporal coherence length is again used as an input to the splitter 32 of the interferometer. The depth information is obtained by measuring the spectral density in the detection arm of the interferometer using a spectrometer 34 in the form of a line array detector. The interference beam is dispersed by a diffraction grating 36 and the individual wavelength components are detected by an array detector.

In SD-OCT systems, the path difference remains fixed and can be assumed to be zero without loss of generality. The array detector 34 serves to digitize the received spectrum in frequency. The main advantage of the SD-OCT system is that the entire depth profile (A-Scan) is measured from a single spectrum with no mechanical scanning of the reference path. This permits faster acquisition of A-scans using a line scan CCD array. The use of a fast spectrometer has made video-rate imaging possible with this technique. High-speed acquisition without any moving parts minimizes any distortion in the OCT images due to motion in the sample. In this arrangement, point illumination and OCT is used to perform a 2D cross-sectional scan.

Static optics 37 is provided, comprising a lens in the illumination path and/or detection path. A processor 39 is provided for performing the image analysis and reconstruction.

Fig. 3 shows the received interference signal at the linear array detector.

The invention is based on the combination of multiple images taken with different relative positions between the imaging system and the tissue, such as caused by longitudinal movement of the breast, in the case of a breast imaging application. However, this relative displacement is obtained without using any scanning optics, but instead makes use of movement of the tissue induced by an applied pressure. In preferred implementations, this tissue movement is already present because the imaging system is integrated into a system that already makes use of pressure application to the tissue.

One example of particular interest is incorporating an imaging function into a breast pump. The breast pump applies a cyclic variable pressure to the breast, resulting in breast movement and deformation. For this purpose, the breast pump has a mechanical or pump actuator (hydraulic, pneumatic etc.) that supplies a well-defined vacuum profile to create a cyclic or periodic skin tissue deformation/motion which is moved into the optical plane(s) of the static optical system. There is typically longitudinal movement of the breast, parallel to the nipple axis, but also breast shape deformation (and hence tissue movement) perpendicular to that axis.

When incorporated into a breast pump, the imaging system is able to create images particularly of nipple tissue, both superficial and subsurface, during breast pumping. By using static optics, there are no parts which are needed for area scanning such as actuators, galvo scanning mirrors or resonant scanners. The imaging function may be integrated into existing breast pumps such as standard and wearable breast pumps.

The image processing by the processor 39 involves storing the captured images in memory and storing image coordinates for reconstructing the images. An image formation unit is then used to stitch the individual images to create a 1D, 2D or 3D reconstruction depending on the nature of the originally captured images (examples are below).

For the example of integration into a breast pump, the device geometry and the pump motion is well known from the device design. This can lead to a well-defined scanning motion of the nipple within the field of view of the optical imaging system. Reconstruction can be then done from the image sequence itself using well-known image reconstruction methods such as registration. Here a requirement might be that the consecutive images need to be partially overlapping. Thus, as long as there is a processor which stores relative nipple positions when the nipple moves into the depth of field of the optical imaging system during the pumping cycle, the images can be combined to create reconstructed images.

Different parts of the breast tissue will pass the field of view of the imaging system, and the tissue is also deformed. This means that different images cannot strictly be combined for longitudinal reconstruction of the duct. The overlap of images enables the reconstruction, for example using the thickness of the adipose tissue (tissue between the ducts) for correction for tissue deformation, or making use of multiple cycles in time.

During the tissue deformation or movement, only a section or part of the tissue moves into the focal plane of the imaging system. Thus line, area, or volume images can be reconstructed from a sequence of multiple images acquired during application of a variable pressure, such as the vacuum profile used for milk pumping.

In addition to creating a stitched image, image analysis may be used to monitor the condition of the nipple and its stretching while feeding or expressing. This can give insights into the milk ejection reflex or indicate possible nipple damage. Blockages of the nipple can be detected. Furthermore, monitoring the nipple while milk is flowing or, just after the milk ejection reflex but without a milk flow, can be used to identify which milk duct is blocked and hence can be used for the detection of blocked ducts in the breast, and mastitis prevention.

The example of Fig. 2 constructs a 2D image (in a plane perpendicular to the nipple axis, shown dotted) by combining line images at different nipple positions.

However, many different types of subsurface optical measurement may be employed, making use of a static optical arrangement in combination with the known nipple motion to create a 1D (line) or 2D (sheet) or 3D (volume) image without the requirement to actuate the optics arrangement.

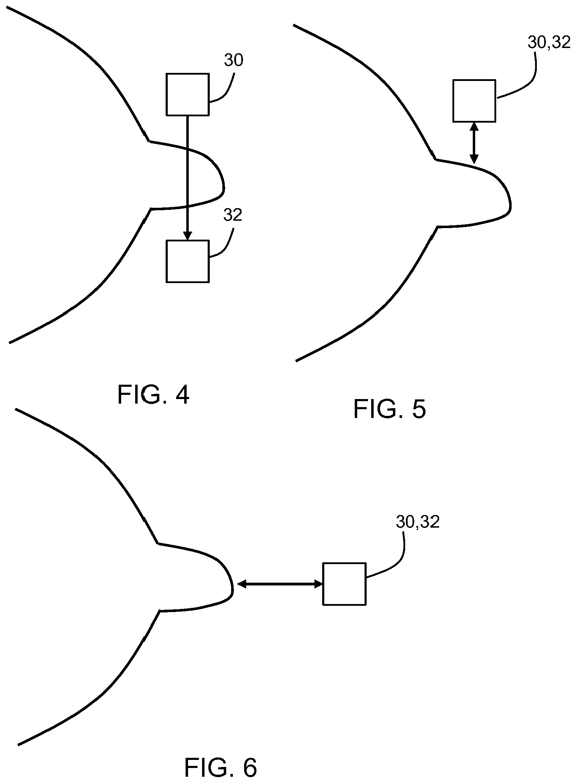

Fig. 4 shows one general arrangement with the light source 30 and detector 32 on opposite lateral sides of the nipple. The light detector is for detecting light which has being transmitted through the full volume of the tissue. This provides trans-illumination. The nipple stretches along its axis during breast pumping so that different nipple portions are imaged at different times.

Fig. 5 shows another general arrangement with the light source 30 and detector 32 on the same lateral side of the nipple. The light detector is for detecting light which has being reflected by the nipple. This provides epi-illumination.

Fig. 6 shows another general arrangement with the light source 30 and detector 32 in front of the nipple.

Some examples will now be explained in more detail.

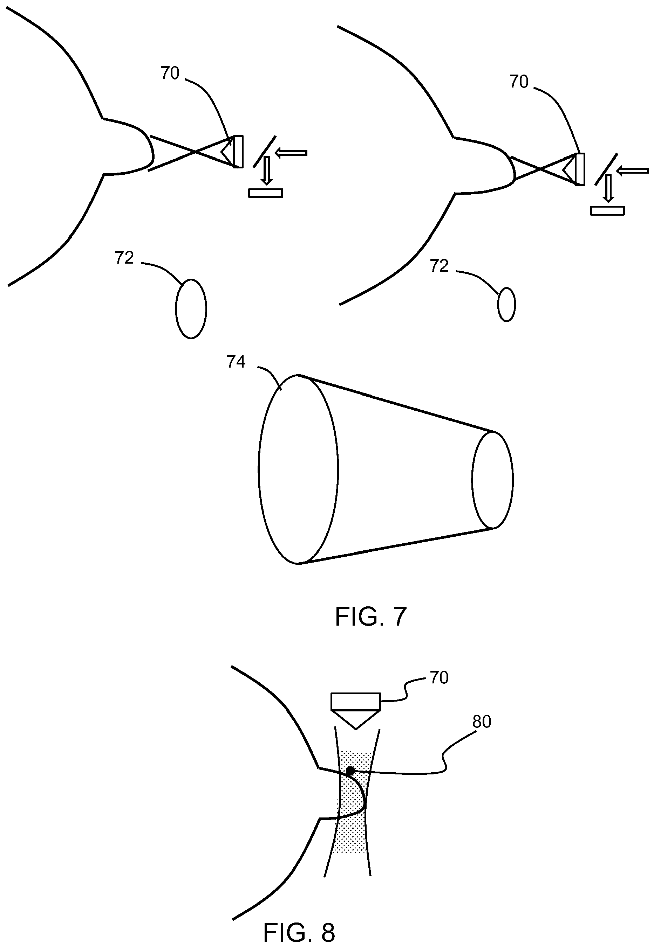

A first example is shown in Fig. 7, wherein the static optics comprises an axicon lens 70 to create a ring of illumination. The axicon lens and its ring illumination is used in near infrared, NIR, and/or infrared, IR, imaging to obtain a 2D subsurface IR / NIR image.

The optical imaging system is in front of the nipple. The distance of the nipple to the optical imaging system influences the size of the captured image, as represented by the ellipses 72. The nipple movement enables a cone volume 74 to be imaged. The position of the axicon lens determines the size of the cone. The ring formed by the axicon lens remains in focus regardless of distance from the lens. The apex angle of the axicon lens determines the tapering of the cone. When using epi-illumination, part of the axicon lens is used for illumination and part is used for imaging. The image plane is formed with an extended depth of field.

The static optics may also make use of an axicon lens to create an extended depth of focus. An axicon lens has a higher depth of focus compared to a conventional spherical lens - the depth of focus of an axicon lens can be as high as 6mm which is suitable for the variable distance between the lens and the nipple during breast pumping action.

Fig. 8 shows the depth of focus 80 of the axicon lens.

Instead of line illumination as discussed above, the imaging system may make use of a fixed focus illumination spot, combined with unpolarized (scattering) detection. This may be used in an epi-illumination system or a trans-illumination system, with point illumination and point detection to detect signals reflected and scattered off the nipple during pumping. It may instead be used in a trans-illumination system, with point illumination and point detection to detect signals scattered and transmitted through the nipple during pumping.

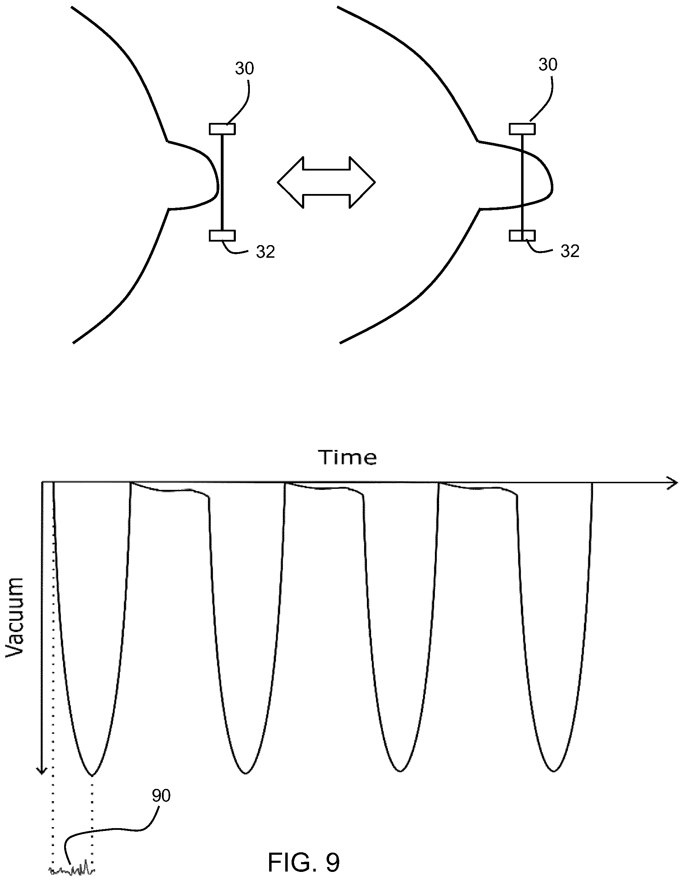

Fig. 9 shows how the detection signal of a 1D (point) detector may vary over time in synchronism with the pumping action. An example signal is shows as 90.

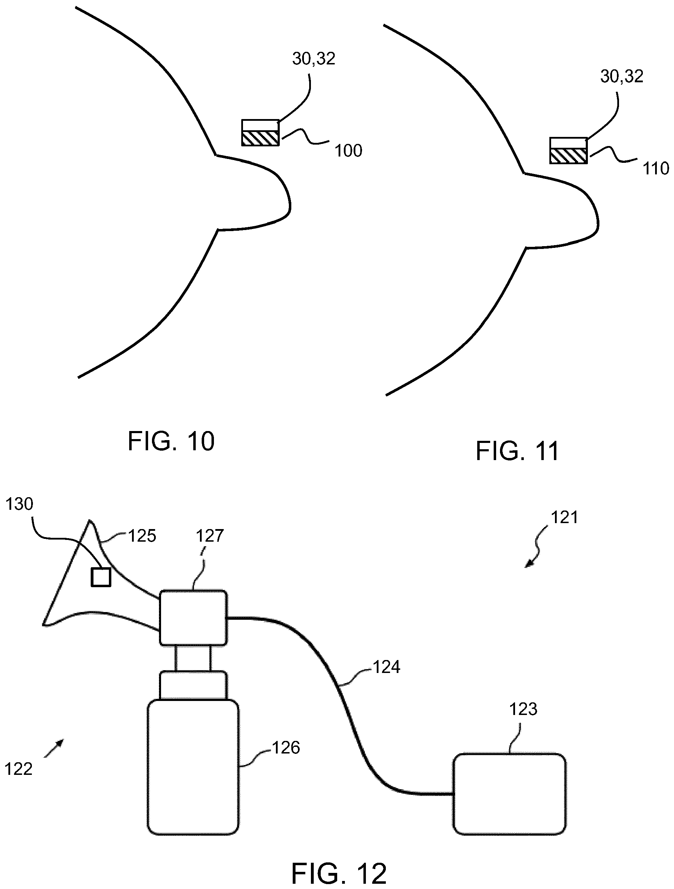

A fixed focus illumination spot may also be combined with polarization detection. As shown in Fig. 10, polarization optics 100 are used in the illumination and detection paths to remove the surface glare and specular reflection of the nipple and to capture scattered photons and sub-surface features more deeply. This approach may be used in both trans- and epi- illumination and detection system. This approach can be combined with the other approaches above.

A fixed focus illumination spot may also be combined with fluorescence detection. As shown in Fig. 11, a fluorescence filter 110 may be provided in front of the detection optics and used to capture the spectral properties of the nipple and sub-surface features. This approach may be used in both trans- and epi- illumination and detection system. This approach can be combined with the other approaches above.

A fixed focus illumination spot may also be used with hyperspectral detection. Spectrometer optics may in this case be used in the detection to capture the spectral properties of the nipple and sub-surface features. This approach can again be used in both trans- and epi- illumination and detection systems. The captured signal (e.g. 90 in Fig. 9) then comprises a set of signals at different wavelengths. The resulting image is thereby a reflectance or transmission spectrum of each pixel along the line, which can be constructed into a 2D spectrogram.

Another example of a static optical system is a cylindrical lens to create a line illumination, and a line detector may then be used.

In the examples above, the optical detection system is entirely static, i.e., the position of the system is fixed related to a reference point, e.g. a nursing bra in which the wearable breast pump is being worn.

However, the optical imaging system, or the whole tissue imaging system, may be movable. For example, it may be mounted at different angular orientations around the nipple. In this way, multiple scans may be combined. For example, multiple direction OCT B scans may be recorded, i.e. multiple 2D cross-sectional scans can be created.

Using line illumination and OCT, a 3D volumetric scan may be performed. Different 3D scans from different angles can create full nipple 3D imaging and increase the quality of scans.

Circular B scans may be combined in a similar manner instead of line scans.

Another option is to perform elastography from a line-illumination system or multiple direction OCT. Swollen or hardened or stiff milk gland lobes and lactiferous ducts can be early signs of infection or mastitis but are often difficult to reliably detect in conventional OCT. Superposing strain fields on structural OCT images may be used to enhance image contrast and diagnostic value. For example, subsurface elastic properties of the nipple can have impact on the flexibility or sensitivity of the nipple. As such, elastography can be used to give more understanding on how to express milk in a more optimal way, and how to apply vacuum.

Information about the elastic properties is also relevant for predicting the onset of nipple cracks (nipple damage) during breastfeeding or milk expression. Elastic properties of the nipple are likely to affect the chance of skin damage.

OCT-elastography may be performed based on phase-sensitive OCT, to determine sub-surface strain, strain rates or stiffness properties of nipples. The phase difference before and after loading (e.g., successive B-scans at different vacuum pump levels) may be used to derive local displacement, strain, and stiffness field. In a preferred implementation of this concept, a fixed focus circular (concentric ring) axion lens is used. In the reference arm, a similar axicon lens is used to create a reference signal, synchronized with the vacuum profile. The interference between the sample and the reference signal is recorded by a ring detector or a camera, and OCT images and phase sensitive OCT images are recorded.

OCT images can also be used to indicate flow velocity, direction, and volume (hence identify potential clogging) in the nipple ducts by analyzing the doppler signal of repeated or adjacent 2D images (B-scans).

Some examples above can be used to create repeated B-scans of the nipple tissue during scanning. The challenge is forming a Doppler signal of the milk flowing through the ducts with a periodically moving nipple, as the movement signal is then a sum of nipple motion and milk flow. The doppler signal can then be recovered using the expression 0.5*(maximum signal + minimum signal).

The invention is of particular interest for mother's health monitoring during breastfeeding, as a stand-alone clogged duct detector or integrated e.g. in breast pumps.
When incorporated into a breast pump, it may be used to provide personalized feedback on optimal milk expression, and optimal application of vacuum.

Fig. 12 shows a known breast pump system 120, comprising an expression unit 122 and a drive arrangement for controlling the expression function of the breast pump.

In the example shown, the drive arrangement comprises an electric pump arrangement 123 which is connected via a tube 124 to the expression unit 122. The pump arrangement includes various components in addition to a pump impeller and the pump motor, so may be considered to be a general operating unit.

The expression unit 122 is formed with a main body 127, a funnel 125 (known as a breast shield) for receiving a breast of a user and a milk collection container 126 for collecting the expressed milk. The funnel 125 and the container 126 are connected to the main body 127. The main body 127 comprises a vacuum chamber. A flexible membrane known as the diaphragm is located in the vacuum chamber. The diaphragm prevents expressed milk from flowing into the tube 124 leading to the pump arrangement unit 123.

The operating unit, including the pump arrangement 123, may instead be directly mounted and connected to the main body 127. In this case, the diaphragm prevents expressed milk from flowing directly into the pump arrangement 123.

The pump arrangement 123 comprises a controller, a power source, a motor and a vacuum pump (or a displacement pump). The controller controls the operation of the power source, motor and vacuum pump to apply a vacuum to the diaphragm located in the main body 127 so that it deforms. The diaphragm deforms to create a vacuum in the funnel 125, when it is sealed to the breast, which in turn applies a vacuum to the breast which enables milk to be expressed.

The vacuum is applied to the breast at intervals. That is, a pressure differential is applied on a cyclic basis. After a vacuum has been established, the pressure from the vacuum is released by the use of the solenoid valve which is temporarily opened. A solenoid valve is used to open and close an air passage that connects the vacuum side of the vacuum pump to ambient air such that when the solenoid valve is closed, the vacuum pump generates a vacuum in the expression unit which enables milk to be expressed from the breast of a user. When the solenoid valve is opened, the vacuum generated by the vacuum pump is released as ambient air flows towards the vacuum or negative pressure created by the vacuum pump such that the pressure exerted on the breast of a user is partially or completely reduced.

This is a basic description of the known operation of a standard breast pump system. There are also wearable breast pumps, which are for example for mounting within a feeding bra. All of the drivetrain components described above are then formed within an outer enclosure which fits over the breast. A top part contains the drivetrain (pump, motor, controller) and a bottom part forms the collection container. This enables breast pumping to be performed more discretely and leaves the hands free to do other things.

The tissue imaging system 130 of the invention is for example mounted in the funnel 125. It may instead be mounted outside the funnel with an optical interface between the imaging system and the funnel. The imaging system may even be remote from the funnel (e.g. at the pump unit), with an optical coupling between the funnel and the imaging system, to relay the illumination light to the nipple area and collect the light for the detection.

The invention is also of interest more generally for tissue analysis and imaging.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processor may be implemented by a single processor or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A system for imaging a tissue during application of a variable pressure to the tissue which induces tissue deformation or movement, comprising:
an optical imaging system comprising a light source (30), static optics (37) for illuminating the tissue and/or collecting the photons that have interacted with the tissue, and at least one light detector,
wherein a distance between the optical imaging system and the tissue is variable in dependence on the tissue deformation or movement such that different portions of the tissue lie within the depth of field of the imaging system at different times during the application of the variable pressure; and
a processor (39) for processing images captured by the optical imaging system to construct a combined image which includes said different portions of the tissue.

2. The system of claim 1, wherein the system is for imaging breast tissue.

3. The system of claim 2, wherein the optical imaging system is configured to image the breast from in front of the nipple or from a side of the nipple.

4. The system of any one of claims 1 to 3, wherein the static optics comprises an axicon lens for generating a ring of illumination.

5. The system of any one of claims 1 to 4, wherein the optical imaging system is adapted to generate a line profile.

6. The system of any one of claims 1 to 3, wherein the static optics comprises a lens for generating a focused illumination spot and the light detector is for detecting scattered, reflected light.

7. The system of claim 6, wherein the optical imaging system further comprises one or more polarizers.

8. The system of any one of claims 1 to 3, wherein the static optics comprises a lens for generating a focused illumination spot and the light detector is for detecting light which has being transmitted through the full volume of the tissue.

9. The system of claim 6 or 8, wherein the optical imaging system further comprising a fluorescence filter.

10. The system of any one of claims 1 to 3, wherein:
the light source is a broadband light source, and the detector is a hyperspectral detector; or
the light source is a wavelength sweeping light source and the detector is a single point detector.

11. The system of any one of claims 1 to 3, wherein the processor is for processing captured images from different angular orientations relative to the tissue to create a 2D or 3D volumetric scan.

12. The system of any one of claims 1 to 11, wherein the processor is further configured to determine elastic or viscoelastic properties of the nipple.

13. The system of any one of claims 1 to 12, wherein the processor is further configured to provide Doppler flow imaging.

14. A breast pump comprising:
a pressure source for generating a cyclic pressure; and
a breast shield for applying the cyclic pressure to the breast; and
the tissue imaging system of any one of claims 1 to 13 for detecting milk duct blockage.

15. A method for imaging a tissue, comprising:
illuminating the tissue and detecting photons that have interacted with the tissue using an optical imaging system thereby to generate captured images;
applying a variable pressure to the tissue which induces tissue deformation or movement, wherein a distance between the optical imaging system and the tissue is variable in dependence on the tissue deformation or movement such that different portions of the tissue lie within the depth of field of the imaging system at different times during the application of the variable pressure; and
processing images captured by the optical imaging system to construct a combined image which includes said different portions of the tissue.
